# EUROPÄISCHE PATENTSCHRIFT

(11) **EP 0 659 374 B1**
(45) Veröffentlichungstag und Bekanntmachung des Hinweises auf die Patenterteilung: **14.06.2000**
(21) Anmeldenummer: 94119978.8
(22) Anmeldetag: 16.12.1994
(51) Int. Cl.: A61F 7/00, A47G 9/02

(54) **Decke zum Wärmen oder Kühlen von Patienten**
Cover for heating or cooling of patients
Couverture pour réchauffer ou rafraîchir les patients

(30) Priorität: 24.12.1993 DE 9319969 U
(43) Veröffentlichungstag der Anmeldung: 28.06.1995
(73) Patentinhaber: Mallinckrodt Medical GmbH, D-53761 Hennef/Sieg (DE)
(72) Erfinder: Löhlein, Christian, Dr. med., NL-6294 NJ Vijlen (NL)
(74) Vertreter: Preissner, Nicolaus, Dipl.-Ing.

(56) Entgegenhaltungen:
- EP-A- 0 305 888
- EP-A- 0 311 336
- US-A- 2 701 885
- US-A- 4 777 802
- US-A- 4 867 230
- US-A- 4 959 877
- US-A- 5 304 213

## Beschreibung

Die Erfindung betrifft eine Decke zum Wärmen oder Kühlen von Patienten, insbesondere zum Schutz vor Auskühlung oder zur Wiedererwärmung bereits ausgekühlter Patienten bei oder nach operativen Eingriffen, mit einem Innenhohlraum, der durch eine luftundurchlässige Oberseite und durch eine luftdurchlässige Unterseite begrenzt ist, wobei der Innenhohlraum mindestens einen Schlauchstutzen zum Anschluß eines Luftschlauches aufweist.

Aus der US 4,777,802 A ist eine Decke zum Wärmen oder Kühlen bekannt, die für die übliche Benutzung auf einem Bett konzipiert ist. Die Decke weist einen Innenhohlraum auf, der durch eine luftundurchlässige Oberseite und eine luftdurchlässige Unterseite begrenzt ist. Die Oberseite kann als Gewebeschicht mit einem eng gewebten Gewebematerial oder auch aus Latex hergestellt sein. Die Unterseite ist aus einem luftdurchlässigen Gewebe, insbesondere Taft, hergestellt. Angaben zur Waschbarkeit, insbesondere zur Desinfizierbarkeit der zum Einsatz kommenden Materialien sind der US-Druckschrift nicht zu entnehmen.

Weiterhin sind für die Anwendung bei Patienten konzipierte Wärmedecken bekannt, die eine Auskühlung des Patienten während oder nach Operationen verhindern sollen. Bei Operationen wird hierbei die Wärmedecke auf einen nicht der Operation unterliegenden Körperbereich des Patienten aufgelegt. In den Innenhohlraum der Wärmedecke, der durch eine luftundurchlässige Oberseite und durch eine mit Perforationen versehene luftdurchlässige Unterseite begrenzt ist, wird Warmluft zugeführt. Durch die an der Unterseite vorgesehenen Perforationen gelangt die Warmluft zu der Körperoberfläche des Patienten. Die bekannte Wärmedecke besteht aus einem polyesterverstärkten Papierfasergemisch. Die Wärmedecke ist nur für den einmaligen Gebrauch vorgesehen und muß nach Anwendung entsorgt werden. Dies führt einerseits zu einer Umweltbelastung und ist andererseits mit Entsorgungskosten verbunden.

Hiervon ausgehend liegt der Erfindung die Aufgabe zugrunde, eine Decke der eingangs genannten Art vorzuschlagen, die zur Anwendung bei Patienten, insbesondere im OP-Bereich, mehrfach verwendbar ist.

Zur Lösung dieser Aufgabe ist bei einer Decke der eingangs genannten Art erfindungsgemäß vorgesehen, daß die Oberseite und die Unterseite der Decke aus einem mehrfach wasch- und desinfizierbaren Gewebe bestehen, wobei die Oberseite aus einem luftundurchlässigen mit einer Polyurethan- und/oder Silikonbeschichtung beschichteten Gewebe, insbesondere einem Kunstfasergewebe, und die Unterseite aus einem diffus luftdurchlässigen Gewebe hergestellt sind, wobei die Decke zur Anwendung bei Patienten, insbesondere im OP-Bereich, mehrfach gebrauchbar ist.

Ein wesentlicher Vorteil der erfindungsgemäßen Decke liegt darin, daß sie wasch- und und desinfizierbar ist, und somit für den Mehrfachgebrauch konzipiert ist. Die verwendeten Materialien gestatten eine Sterilisation der erfindungsgemäßen Decke. Hierdurch entfällt die Entsorgung nach dem einmaligen Gebrauch der Decke, was einerseits zu einer Reduzierung der Umweltbelastung und andererseits zu einer Verminderung der Entsorgungskosten führt. Die Verwendung eines textilen Gewebes hat darüber hinaus den weiteren Vorteil, daß die Decke trotz hoher Reißfestigkeit sehr leicht ist und eine hohe Körperverträglichkeit aufweist. Durch die Verwendung eines diffus luftdurchlässigen Gewebes an der Unterseite der Decke wird eine besonders gleichmäßige Verteilung der eingeleiteten Luft und eine gute Körperverträglichkeit erzielt.

Vorteilhafte Ausgestaltungen der Erfindung ergeben sich aus den Unteransprüchen.

Vorteilhaft ist die Oberseite aus einem mit der Beschichtung verschenen Polyamidgewebe hergestellt. Ein derartiges Polyamid- oder Nylongewebe ist prinzipiell bekannt und wird beispielsweise von der Firma VERSEIDAG-Industrietextilien GmbH, Kempen, unter der Bezeichnung Polyant°-Aircloth° vertrieben. Bisher wurde ein derartiges Material zur Herstellung von Spinnaker-Segeln verwendet. Auf Grund der Verwendung von 100 Prozent hochfestem Nylon zeichnet sich dieses Polyamidgewebe durch eine hohe Gewebefestigkeit aus. Das effektive Gewicht beträgt 46 bis 48 Gramm pro Quadratmeter. Durch die luftundurchlässige Beschichtung wird erreicht, daß dieses Polyamidgewebe bei 20 mm Wassersäule eine Luftdurchlässigkeit von Null aufweist.

In weiterer Ausgestaltung wird vorgeschlagen, daß die Unterseite aus einem diffus luftdurchlässigen Polyamidgewebe besteht. Zweckmäßig kommt ein diffus luftdurchlässiges Polyamid- oder Nylongewebe zum Einsatz, das ein Flächengewicht von 53 g/m² aufweist. Bei einer Gewebedichte von etwa 104 µm weist dieses Material für die Unterseite der Decke eine hohe Reißfestigkeit auf.

Die diffus luftdurchlässige Unterseite weist vorteilhaft eine Luftdurchlässigkeit von 78 bis 107 Liter/dm² x Minute auf. Hierdurch wird eine besonders gleichmäßige Verteilung der in die Decke eingeleiteten Luft erzielt. Aufgrund der Leichtigkeit des Materials wird eine sehr hohe Körperverträglichkeit erreicht.

In weiterer Ausgestaltung kann die Oberseite eine die Wärme reflektierende Beschichtung aufweisen, insbesondere eine Aluminiumbeschichtung. Hierdurch ist eine besonders gezielte Erwärmung bestimmter Körperpartien möglich.

Vorteilhaft besteht der Schlauchstutzen aus einem luftdichten, wasch- und und desinfizierbaren Material.

Der Schlauchstutzen kann eine Anschlußöffnung aufweisen, deren Öffnungsquerschnitt einstellbar ist. Hierdurch ist eine Anpassung an verschiedenartige Luftschläuche möglich.

Vorteilhaft kann die Anschlußöffnung einen Saum aufweisen, in dem ein Band zum Einstellen des Öffnungsquerschnittes geführt ist.

An der dem Schlauchstutzen gegenüberliegenden Innenseite des Innenhohlraumes kann eine Verstärkungslage vorgesehen sein. Hierdurch wird ein zu starker lokaler Luftdurchtritt unterbunden.

Insbesondere bei einer zur Erwärmung des Oberkörpers konzipierten Ausführungsform ist vorteilhaft vorgesehen, daß der Außenrand der Decke mit einer Vielzahl von beabstandeten Haltebändern versehen ist.

Weiterhin kann der Außenrand der Decke mit einem senkrecht abragenden Kopftuch versehen sein, das vorteilhaft zweigeteilt ausgebildet ist. Ein derartiges Kopftuch kann im Gebrauch eine Auskühlung des Kopfbereiches verhindern.

In weiterer Ausgestaltung sind dem Kopftuch gegenüberliegend Haltebänder vorgesehen, die eine größere Länge als die anderen Haltebänder aufweisen. Hierdurch ist eine Fixierung der Wärmedecke am Rippenbogen ohne Verwendung von Klebband, insbesondere Heftpflaster, möglich.

Nachfolgend soll die Erfindung anhand von Ausführungsbeispielen, die in schematischer Weise in der Zeichnung dargestellt sind, näher erläutert werden. Hierin zeigen:
- Fig. 1: eine Draufsicht auf eine erste erfindungsgemäße Ausführungsform, die als Oberkörper-Wärmedecke ausgelegt ist;
- Fig. 2: einen Schnitt längs der Linie II-II in Fig. 1;
- Fig. 3: eine vergrößerte Darstellung der Einzelheit III in Fig. 1, und
- Fig. 4: eine weitere erfindungsgemäße Ausführungsform, die als Ganzkörper-Wärmedecke ausgelegt ist.

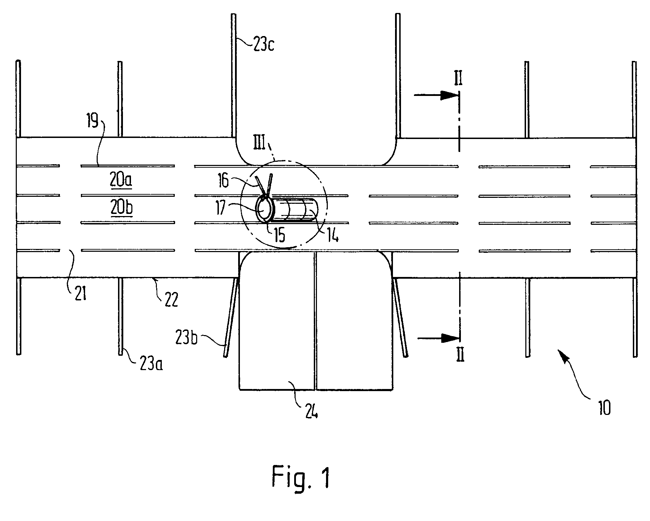

Die in Fig. 1 in Draufsicht dargestellte Wärmedecke 10 ist für Operationen im Bauchraum, der Hüfte oder den unteren Extremitäten geeignet und wird an dem Oberkörper und an den Armen des Patienten aufgebracht. Die Wärmedecke 10 besteht aus einem wasch- und desinfizierbaren Kunstfasergewebe und ist somit für den mehrfachen Gebrauch geeignet. Die Wärmedecke 10 kann wie andere im OP-Bereich verwendete Textilien sterilisiert werden.

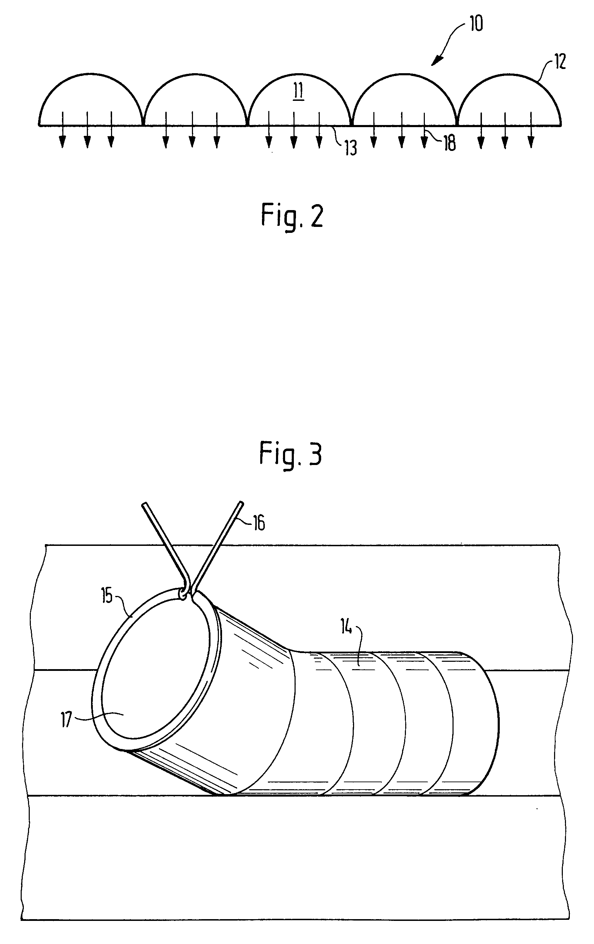

Wie aus Fig. 2 hervorgeht, die einen Schnitt längs der Linie II-II in Fig. 1 zeigt, weist die Wärmedecke 10 einen Innenhohlraum 11 auf, der durch eine Oberseite 12 und eine Unterseite 13 begrenzt ist. Die Oberseite 12 der Wärmedecke 10 besteht aus einem luftdichten Polyamid- oder Nylongewebe, das eine Polyurethanbeschichtung aufweist. Ein derartiges Gewebe ist prinzipiell bekannt und wurde bisher insbesondere zur Herstellung von Spinnaker-Segeln verwendet. Diese Nylongewebe zeichnet sich neben ihrer hohen Luftundurchlässigkeit durch hohe Reißfestigkeit, geringes Gewicht und eine gute Körpervertraglichkeit aus.

An der Unterseite 13 der Wärmedecke 10 kommt ein diffus luftdurchlässiges Polyamid- oder Nylongewebe zum Einsatz. Auch dieses Nylongewebe, das prinzipiell bekannt ist, zeichnet sich durch geringes Gewicht, hohe Reißfestigkeit und durch seine angenehme Körperverträglichkeit aus. Da die luftdurchlässige Unterseite als Gewebe mit gleichmäßiger Gewebestruktur vorliegt, wird eine besonders gleichmäßige Luftverteilung erreicht.

Der Innenhohlraum 11 ist in eine Vielzahl von Kammern 20a, 20b unterteilt, die durch eine Vielzahl von Nähten 19 voneinander abgetrennt sind. Zwischen den Kammern 20a, 20b sind Verbindungsöffnungen 21 vorgesehen.

Wie aus den Figuren 1 und 3 ersichtlich ist, ist an der Oberseite 12 der Wärmedecke 10 ein Schlauchstutzen 14 vorgesehen, der aus einem luftdichten, wasch- und desinfizierbaren Textilgewebe besteht. Der Schlauchstutzen 14 ist als Textilschlauch ausgebildet und an die Oberseite 12 der Wärmedecke 10 angenäht. Eine Anschlußöffnung 17 ist durch einen ringförmigen Saum 15 begrenzt, in dem ein Band 16 geführt ist. Hierdurch ist die Anpassung an verschiedene Durchmesser eines Warmluftschlauches in einfacher Weise möglich.

Wie aus der Zeichnung nicht hervorgeht, ist im Bereich des Schlauchstutzens 14 an der Innenseite der Unterseite 13 eine Verstärkungslage vorgesehen. Hierdurch wird ein zu starker lokaler Luftdurchtritt verhindert.

Ein Außenrand 22 der Wärmedecke 10 ist mit einer Vielzahl von beabstandeten Haltebändern 23a, 23b, 23c versehen. Die Haltebänder 23a, 23b, 23c sind ebenfalls aus einem wasch- und desinfizierbaren Textilgewebe, insbesondere Nylongewebe, hergestellt.

Die Haltebänder 23a, 23b, 23c dienen der Fixierung der Wärmedecke 10 am Oberkörper des Patienten.

Im mittleren Bereich der Wärmedecke 10 ist ein Kopftuch 24 vorgesehen, das zweigeteilt ausgebildet ist. Das Kopftuch 24, das ebenfalls aus einem Kunstfasergewebe besteht, ist an den Außenrand 22 der Wärmedecke 10 angenäht.

Die dem Kopftuch 24 gegenüberliegenden Haltebänder 23c sind länger als die übrigen Haltebänder 23a, 23b ausgebildet. Hierdurch wird erreicht, daß auch ohne zusätzliche Klebebänder, insbesondere Heftpflaster, eine Fixierung der Wärmedecke 10 am Rippenbogen möglich ist.

Wie aus der vorstehenden Detailbeschreibung der Wärmedecke 10 hervorgeht, sind sämtliche Teile aus wasch- und desinfizierbarem Material hergestellt. Somit ist die Wärmedecke 10 für einen Mehrfachgebrauch geeignet.

Bei der Anwendung der Wärmedecke 10 wird diese auf den Oberkörper und die Arme des Patienten aufgelegt und mittels der Haltebänder 23a, 23b, 23c fixiert. In den Schlauchstutzen 14 wird dann ein in der Zeichnung nicht dargestellter Warmluftschlauch eingeführt, der mit einem Warmluftgebläse verbunden ist. Der Durchmesser der Anschlußöffnung 17 kann mittels des Bandes 16 eingestellt werden. Über den Schlauchstutzen 14 gelangt die Warmluft in den Innenhohlraum 11, wodurch dieser in der in Fig. 2 dargestellten Weise aufgeblasen wird. Durch die luftdurchlässige Unterseite 13 gelangt die Warmluft in Richtung der Pfeile 18 an den Körper des Patienten. Das zum Einsatz kommende Nylongewebe, das diffus luftdurchlässig ist, gewährleistet eine gute Luftverteilung. Somit wird die eingebrachte Warmluft gleichmäßig auf den Körper des Patienten verteilt.

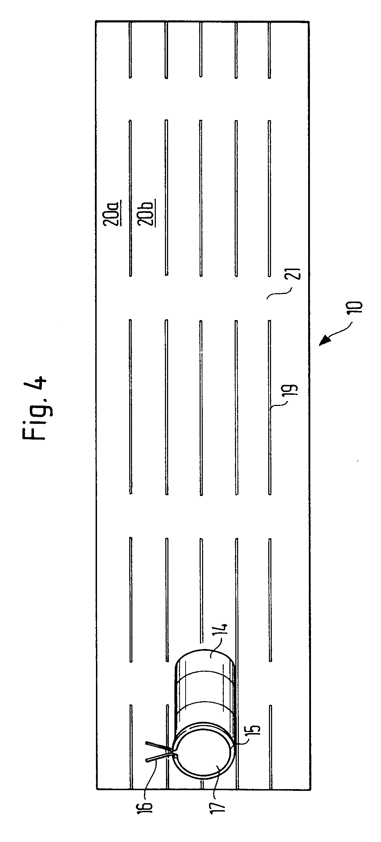

Fig. 4 zeigt eine weitere Ausführungsform der Erfindung, die als Ganzkörper-Wärmedecke ausgebildet ist. Nachfolgend werden die bereits eingeführten Bezugszeichen für gleiche oder funktionsgleiche Teile verwendet.

Die Wärmedecke gemäß Fig. 4 besitzt einen prinzpiell ähnlichen Aufbau wie die Wärmedecke gemäß den Fig. 1 bis 3. Auch diese Wärmedecke ist aus einem wasch- und desinfizierbaren Material, insbesondere einem Kunstfasergewebe hergestellt. Wie aus der Draufsicht gemäß Fig. 4 hervorgeht, besitzt die Wärmedecke 10 Rechteckform. Einen Innenhohlraum der Wärmedecke 10 ist durch eine Vielzahl von Nähten 19 in Kammern 20a, 20b unterteilt, die über Verbindungsöffnungen 21 in Verbindung stehen. An der Oberseite der Wärmedecke 10 ist ein Schlauchstutzen 14 vorgesehen, der eine Anschlußöffnung 17 für einen nicht dargestellten Warmluftschlauch aufweist. Mittels eines in einem Saum 15 geführten Bandes 16 ist der Durchmesser der Anschlußöffnung 17 einstellbar.

Die vorstehend beschriebenen Wärmedecken sind vollständig aus einem wasch- und desinfizierbaren Material hergestellt und somit für den mehrfachen Gebrauch konzipiert.

Einen prinzipiell gleichartigen Aufbau weisen in der Zeichnung nicht dargestellte Kühldecken auf. Bei diesen Decken wird über den Luftschlauch Kaltluft zugeführt, die über die luftdurchlässige Unterseite abströmt.

Die bei der Verwendung von Einmaldecken bekannten Entsorgungsprobleme und Umweltbelastungen treten bei den erfindungsgemäßen Decken somit nicht auf. Weiterhin weisen die erfindungsgemäßen Decken eine luftdurchlässige Unterseite auf, die die zugeführte Luft diffus und somit besonders gleichmäßig verteilt. Darüber hinaus ist das zum Einsatz kommende Kunstfasergewebe besonders leicht und sehr körperverträglich.

## Patentansprüche

1. Decke zum Wärmen oder Kühlen von Patienten, insbesondere zum Schutz vor Auskühlung oder zur Wiedererwärmung bereits ausgekühlter Patienten bei oder nach operativen Eingriffen, mit einem Innenhohlraum (11), der durch eine luftundurchlässige Oberseite (12) und eine luftdurchlässige Unterseite (13) begrenzt ist, wobei der Innenhohlraum (11) mindestens einen Schlauchstutzen (14) zum Anschluß eines Luftschlauches aufweist, dadurch gekennzeichnet, daß die Oberseite (12) und die Unterseite (13) der Decke (10) aus einem mehrfach wasch- und desinfizierbaren Gewebe bestehen, wobei die Oberseite (12) aus einem luftundurchlässigen mit einer Polyurethan- und/oder Silikonbeschichtung beschichteten Gewebe, insbesondere einem Kunstfasergewebe, und die Unterseite (13) aus einem diffus luftdurchlässigen Gewebe hergestellt sind, wobei die Decke zur Anwendung bei Patienten, insbesondere im OP-Bereich, mehrfach gebrauchbar ist.

2. Decke nach Anspruch 1, dadurch gekennzeichnet, daß die Oberseite aus einem mit der Beschichtung versehenen Polyamidewebe hergestellt ist.

3. Decke nach Anspruch 1 oder 2, dadurch gekennzeichnet, daß die Unterseite (12) aus einem diffus luftdurchlässigen Polyamidgewebe besteht.

4. Decke nach Anspruch 1 bis 3, dadurch gekennzeichnet, daß die Unterseite eine Luftdurchlässigkeit von 78 bis 107 Liter/dm² x Minute aufweist.

5. Decke nach einem der Ansprüche 1 bis 4, dadurch gekennzeichnet, daß die Oberseite (12) eine Wärmestrahlung reflektierende Beschichtung, insbesondere eine Aluminiumbeschichtung, aufweist.

6. Decke nach einem der Ansprüche 1 bis 5, dadurch gekennzeichnet, daß der Schlauchstutzen (14) aus einem luftdichten, wasch- und desinfizierbaren Material besteht.

7. Decke nach einem der Ansprüche 1 bis 6, dadurch gekennzeichnet, daß der Schlauchstutzen (14) eine Anschlußöffnung (17) aufweist, deren Öffnungsquerschnitt einstellbar ist.

8. Decke nach Anspruch 7, dadurch gekennzeichnet, daß die Anschlußöffnung (17) einen Saum (15) aufweist, in dem ein Band (16) zum Einstellen des Öffnungsquerschnittes geführt ist.

9. Decke nach einem der Ansprüche 1 bis 8, dadurch gekennzeichnet, daß an der dem Schlauchstutzen (14) gegenüberliegenden Innenseite des Innenhohlraumes (11) eine Verstärkungslage vorgesehen ist.

10. Decke nach einem der Ansprüche 1 bis 9, dadurch gekennzeichnet, daß der Außenrand (22) der Decke (10) mit einer Vielzahl von beabstandeten Haltebändern (23a, 23b, 23c) versehen ist.

11. Decke nach einem der Ansprüche 1 bis 10, dadurch gekennzeichnet, daß der Außenrand (22) der Decke (10) mit einem senkrecht abragenden Kopftuch (24) versehen ist, das vorteilhaft zweigeteilt ausgebildet ist.

12. Decke nach Anspruch 11, dadurch gekennzeichnet, daß dem Kopftuch (24) gegenüberliegend Haltebänder (23c) vorgesehen sind, die eine größere Länge als die anderen Haltebänder aufweisen.

13. Decke nach einem der Ansprüche 1 bis 12, dadurch gekennzeichnet, daß der Innenhohlraum (11) eine Vielzahl von abgetrennten Kammern (20a, 20b) aufweist, die durch Verbindungsöffnungen (21) miteinander verbunden sind.

## Claims

1. A blanket for warming or cooling a patient, more particularly for protecting against loss of body heat or to compensate loss of body heat during or after an operation, including an inner cavity (11) defined by an air-impermeable top side (12) and an air-permeable underside (13), said inner cavity (11) comprising at least one hose inlet (14) for connecting an air hose, characterized in that said top side (12) and said underside (13) of said blanket (10) are made of a fabric suitable for repeated washing and disinfection, said top side (12) being made of an air-impermeable fabric, more particularly of a synthetic fiber fabric, coated with a polyurethane and/or silicone coating, and said underside (13) being made of a diffused air-permeable fabric, said blanket being intended for repeated use on patients especially in an operating room environment.

2. The blanket as set forth in claim 1, characterized in that said top side (12) is made of a polyamide fabric provided with said coating.

3. The blanket as set forth in claim 1 or 2, characterized in that said underside (13) is made of a diffused air-permeable polyamide fabric.

4. The blanket as set forth in any of the claims 1 to 3, characterized in that said underside (13) features an air permeability of 78 to 107 liters/dm² per minute.

5. The blanket as set forth in any of the claims 1 to 4, characterized in that said top side (12) comprises a coating, more particularly an aluminum coating, reflecting thermal radiation.

6. The blanket as set forth in any of the claims 1 to 5 characterized in that said hose inlet (14) consists of an air-tight material suitable for washing and disinfection.

7. The blanket as set forth in any of the claims 1 to 6, characterized in that said hose inlet (14) comprises a connecting port (17) the throat of which is variable.

8. The blanket as set forth in claim 7, characterized in that said connecting port (17) comprises a hem (15) in which a tape (16) for varying said throat is guided.

9. The blanket as set forth in any of the claims 1 to 8, characterized in that a reinforcement ply is provided at the inner side of said inner cavity (11) opposite said hose inlet (14).

10. The blanket as set forth in any of the claims 1 to 9, characterized in that said outer edge (22) of said blanket (10) is provided with a plurality of retaining tapes (23a, 23b, 23c) spaced away from each other.

11. The blanket as set forth in any of the claims 1 to 10, characterized in that said outer edge (22) of said blanket (10) is provided with an upright hood (24) advantageously configured two-part.

12. The blanket as set forth in claim 11, characterized in that retaining tapes (23c) are provided opposite said hood (24) which are longer than said other retaining tapes.

13. The blanket as set forth in any of the claims 1 to 12, characterized in that said inner cavity (11) comprises a plurality of separate chambers (20a, 20b) interconnected by connecting ports (21).

## Revendications

1. Couverture pour le réchauffement ou le refroidissement de patients, en particulier pour la protection vis-à-vis du refroidissement, ou pour le réchauffement de patients déjà refroidis pendant des interventions opératoires, ou après de telles interventions, comprenant une chambre intérieure (11) délimitée par une face supérieure imperméable à l'air (12) est par une face inférieure perméable à l'air (13), ladite chambre intérieure (11) comportant au moins un manchon tubulaire (14) pour le raccordement d'un tube à air, caractérisée en ce que la face supérieure (12) et la face inférieure (13) de la couverture (10) sont formées par un tissu que l'on peut laver et désinfecter plusieurs fois, la face supérieure (12) étant produite à partir d'un tissu imperméable à l'air pourvu d'un revêtement de polyuréthane et/ou de silicone, en particulier un tissu en fibres artificielles, et la face inférieure (13) étant produite à partir d'un tissu perméable à l'air de manière diffuse, grâce à quoi la couverture est utilisable plusieurs fois pour l'application à des patients, en particulier dans le domaine opératoire.

2. Couverture selon la revendication 1, caractérisée en ce que la face supérieure est produite à partir d'un tissu en polyamide pourvu du revêtement.

3. Couverture selon l'une ou l'autre des revendications 1 et 2, caractérisée en ce que la face inférieure (12) est constituée par un tissu en polyamide perméable à l'air de manière diffuse.

4. Couverture selon les revendications 1 à 3, caractérisée en ce que la face inférieure présente une perméabilité à l'air de 78 à 107 l/dm² x minute.

5. Couverture selon l'une des revendications 1 à 4, caractérisée en ce que la face supérieure (12) comporte un revêtement réfléchissant vis-à-vis du rayonnement thermique, en particulier un revêtement en aluminium.

6. Couverture selon l'une des revendications 1 à 5, caractérisée en ce que le manchon tubulaire (14) est constitué en un matériau étanche à l'air, que l'on peut laver et désinfecter.

7. Couverture selon l'une des revendications 1 à 6, caractérisée en ce que le manchon tubulaire (14) comporte une ouverture de raccordement (17) dont la section d'ouverture est réglable.

8. Couverture selon la revendication 7, caractérisée en ce que l'ouverture de raccordement (17) comprend un ourlet (15) dans lequel est guidée une bande (16) pour le réglage de la section d'ouverture.

9. Couverture selon l'une des revendications 1 à 8, caractérisée en ce qu'il est prévu une couche de renfort sur la face intérieure de la chambre creuse (11) opposée au manchon tubulaire (14).

10. Couverture selon l'une des revendications 1 à 9, caractérisée en ce que la bordure extérieure (22) de la couverture (10) est pourvue d'une pluralité de bandes de maintien (23a, 23b, 23c) écartées les unes des autres.

11. Couverture selon l'une des revendications 1 à 10, caractérisée en ce que la bordure extérieure (22) de la couverture (10) est pourvue d'une toile de tête (24), réalisée avantageusement en deux parties, qui dépasse perpendiculairement.

12. Couverture selon la revendication 11, caractérisée en ce qu'il est prévu des bandes de maintien (23c) à l'opposé de la toile de têtes (24), lesquelles présentent une longueur plus importante que les autres bandes de maintien.

13. Couverture selon l'une des revendications 1 à 12, caractérisée en ce que la chambre intérieure (11) comprend une pluralité de cavités séparées (20a, 20b), lesquelles sont reliées les unes aux autres par des ouvertures de liaison (21).
